Europäisches Patentamt

European Patent Office

Office européen des brevets

(11) Publication number: **0 223 831 B1**

## EUROPEAN PATENT SPECIFICATION

(45) Date of publication of patent specification: **15.07.92**

(51) Int. Cl.5: **A61K 9/50**, A61K 9/42, A61K 31/685

(21) Application number: **86903857.0**

(22) Date of filing: **21.05.86**

(86) International application number: **PCT/US86/01095**

(87) International publication number: **WO 86/06959 (04.12.86 86/26)**

(54) LIPOSOME INHALATION METHOD AND SYSTEM.

(30) Priority: **22.05.85 US 737221**

(43) Date of publication of application: **03.06.87 Bulletin 87/23**

(45) Publication of the grant of the patent: **15.07.92 Bulletin 92/29**

(84) Designated Contracting States: **AT BE CH DE FR GB IT LI LU NL SE**

(56) References cited:
**EP-B- 84 898**            **WO-A-87/01586**
**US-A- 4 011 864**       **US-A- 4 137 914**
**US-A- 4 312 860**       **US-A- 4 414 972**

**H.R. Shepard, Aerosols: Science and Technology, published 1961 by Interscience Publishers, Inc. see pages 17-40, 397-405**

(73) Proprietor: **LIPOSOME TECHNOLOGY, INC. 1050 Hamilton Court Menlo Park, CA 94025(US)**

(72) Inventor: **MIHALKO, Paul, J. 36784 Riviera Drive Fremont, CA 94536(US)**
Inventor: **ABRA, Robert, M. 143 Judson San Francisco, CA 94112(US)**

(74) Representative: **Goldin, Douglas Michael et al J.A. KEMP & CO. 14, South Square Gray's Inn London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### 1. Field of the Invention

The present invention relates to drug delivery by inhalation, and, in particular, to an improved drug delivery system and method of using liposome-entrapped drugs.

### 2. References

The following references are incorporated herein by corresponding number:

1. Hollenbeck, R.G., et al, in "Pharmaceutics and Pharmacy Practice" (Banker, G.S., et al, eds), J.P. Lippincott, Philadelphia (1982), pp. 355-358 .
2. Szoka, F., Jr., et al, Ann Rev Biophys Bioeng (1980), 9:467.
3. Szoka, F., Jr., et al, Proc Natl Acad Sci (USA) (1978) 75:4194.
4. Hollenbeck, R.G., op cit, pp. 382-391.

### 3. Background and Summary

Inhalation provides an effective means for delivering a variety of drugs, including nasal decongestants, drugs useful in the treatment of asthma and other bronchial and pulmonary conditions (reference 1). An obvious advantage of inhalation in treating nasal, bronchial, or pulmonary conditions is the ability to deliver the drug directly to the site of drug action. A related advantage is the rapid onset of the therapeutic effect, compared with other routes of administration, such as intramuscular and oral routes. For drugs which are susceptible to breakdown in the gastrointestinal tract, or which otherwise cannot be administered orally, inhalation may be preferred for a variety of reasons over intravenous or intramuscular injection. Other drugs, such as nitroglycerin, whose primary drug action is systemic, can also be delivered efficiently by inhalation.

In one known method for administering a drug by inhalation, the drug is dissolved in a suitable solvent which can be aerosolized to form a small-particle mist. The drug solution may be aerosolized by a pneumatic or ultrasonic nebulizer, or, more conveniently, by a self-contained nebulizer which is driven by gas pressure from a fluorocarbon propellant. Inhalation of the acrosolized mist, i.e., drawing the mist from the mouth or nose into the respiratory tract, acts to deposit the drug-containing aerosol particles on various sites of the respiratory tract, including the upper nasopharyngeal region, the tracheobronchial region, and the pulmonary region. In the latter region, the drug has the opportunity for rapid absorption into the bloodstream for systemic action.

Also well known in the prior art are inhalation systems in which a drug is administered in particulate form, either as a dry powder or as a micronized suspension in a suitable carrier solvent system. Typically the drug is a water-soluble compound which is suspended in micronized form in a fluorocarbon-type propellant solvent. Following aerosolization, most of the propellant solvent is lost through flash evaporation and replaced by moisture in the respiratory tract, leading to the deposition of hydrated micronized particles.

Both types of inhalation systems mentioned above are based on delivery of the drug in a free form to sites in the respiratory tract. As such, the drug is rapidly utilized and, in the case of pulmonary deposition, taken up systemically at the site of deposition. Because of this rapid drug uptake and utilization, it may be necessary to administer the drug at frequent intervals to maintain a desired therapeutic dose level at the site of action. A related problem is the limitation on the amount of drug that can be administered safely at each inhalation, particularly in the case of a drug which has unwanted systemic side effects. This problem is illustrated by a number of $\beta$-adrenergic agonist type bronchodilators which also produce marked tachycardia. Even at relatively low doses of these drugs, the stimulatory effect of the drug on the heart may be a nuisance to the patient. An additional problem associated with administration of micronized particles is the irritation that these particles may produce in the respiratory tract.

More recently, a liposome inhalation system for administering a drug to the respiratory tract in liposome-entrapped form has been proposed (UK patent application # 2,145,107A. A liposome aerosol is formed by mixing lipid and aqueous components in a two-chamber dispenser, then aerosolizing the mixture by forcing it through a nozzle. The mixing and aerosolizing steps are effective to form a liposome aerosol in which a portion of the drug, which may be contained in either the lipid or aqueous component, is present in liposome-entrapped form. This invention suffers the disadvantage that in situ liposome formation, and particularly the efficiency of drug encapsulation in the liposomes, is highly variable, depending on the degree of mixing of the two components just prior to aerosolization, the relative quantities of two components which are mixed, and possibly temperature and humidity conditions which exist during liposome formation. As a result the relative percentages of free and liposome-encapsulated drug, and even the total amount of drug which is delivered can be quite variable. EP-A-0 084 898 describes an aqueous pharmaceutical preparation containing sodium cromoglycate, an antiallergen, and liposomes. Preferably the amount of sodium cromoglycate in the liposomal phase is from 2 to 35% w/w.

4. Summary of the Invention

It is a general object of the present invention to provide a liposome drug inhalation method and system which substantially overcome problems and limitations associated with prior art devices and methods for administering drugs by the inhalation route.

One specific object of the invention is to provide such method and system for use in delivering a water-soluble, liposome-permeable drug, and particularly a system which can be stored over a long period before use.

Still another object of the invention is to provide a method and system in which the rate of drug-release into the respiratory tract can be selectively controlled, according to the lipid composition of the liposomes encapsulating the drug.

The invention includes a method for moderating the initial (short-term) and extended (long-term) drug-level effects of a drug administered by inhalation. The drug is provided in a form in which it is predomiantly entrapped in the liposomes of a liposome suspension, and the suspension is aerosolized in a form suitable for inhalation. According to one aspect of the invention, the sequestering of the drug predominantly in a slow-release, liposome-encapsulated form largely reduces or eliminates unwanted drug-spiking effects due to rapid systemic uptake of the administered drug. The system thus allows greater amounts of drug to be administered at a single dose, with fewer side effects. In the system, the free drug provides a loading dose, and the liposomes-encapsulated drug provides for sustained release delivery.

According to another feature of the invention, the liposomes are formulated to produce a selected rate of release of entrapped drug, for use in achieving controlled systemic levels of the drug. The drug release half lives, as measured in vitro may range from a half hour or less, for liposomes whose phospholipid acyl chain components are relatively short and/or unsaturated, to six days or more, for liposomes whose phospholipid acyl chain components are relatively long and/or saturated.

The system of the invention includes a suspension of liposomes containing a drug predominantly in entrapped form, and a device for aerosolizing a preselected quantity of the suspension, in a form suitable for inhalation.

Two embodiments of the system are designed for administration of water-soluble drugs, particularly liposome-permeable drugs. In the first, the liposomes are prepared in an aqueous, paste-like suspension containing more than 50% encapsulated water, and therefore, more than 50% encapsulated drug after drug equilibration between inner liposomal and outer bulk phase compartments of the paste. The paste is mixed with at least 1-2 volumes of a delivery medium within a mixing chamber, and aerosolized before drug diffusion across the liposomal barrier substantially reduces the percent of liposome-encapsulated drug. The delivery medium may be an aqueous medium, in which case the diluted liposomes can be aerosolized by pneumatic or ultrasonic energy. Alternatively, the paste may be diluted with a fluorocarbon propellent delivery medium, for use in a self-contained aerosol device.

In a second general embodiment, the liposome suspension in the system is composed of drug-containing liposomes dispersed in a fluorocarbon solvent. The percent liposome-encapsulated drug, which exists in an equilibrated state in the suspension, is relatively high due to the greater partitioning of the drug in the encapsulated aqueous compartment than in the external non-aqueous fluorocarbon medium. The liposomes may be suspended in the fluorocarbon either in dried, particulate form, or as an emulsion of aqueous liposome paste particles. The paste, as just noted, contains the drug predominantly in liposome-encapsulated form. The liposome/propellent suspension can be delivered in metered dose form from a conventional self-contained nebulizer.

These and other objects and features of the present invention will become more fully apparent when the following detailed description of the invention is read in conjunction with the accompanying drawings.

Detailed Description of the Drawings

Figure 1 is a plot showing percent protection against acetylcholine-induced bronchoconstriction in an animal following administration by inhalation of free metaproteranol sulfate (MPS)(open squares) or MPS-encapsulated in liposomes having relatively long (closed circles) or relatively short (open circles) in vitro drug efflux half lives;

Figure 2 is a plot showing percent increase in heart rate in an animal following administration of MPS, as in Figure 1.

Figures 3A and 3B are plots showing percent increase in heart rate in a subject following treatment with free MPS (open squares in the figures), and MPS encapsulated in liposomes with relatively high (3A) and low (3B) rates of drug release; and

Figure 4 shows plasma concentrations of MPS in a subject following administration of free drug (open squares) and drug encapsulated in each of the Figure 3 liposome formulations.

## Detailed Description of the Invention

### I. Liposome Suspension

The liposome suspension of the invention is formulated to contain (a) a selected drug predominantly in liposome-entrapped form, and (b) a lipid composition designed to produce a desired drug-release rate when the suspension is admistered to the respiratory tract. Section IA below considers the relationship between liposome bilayer components and drug-release rates. Section IB describes methods for preparing liposomes containing entrapped drugs. Methods of forming liposome suspensions containing a drug in predominantly liposome-entrapped form are discussed in Sections IC-IE.

### IA. Lipid Components and Drug Release Rates

In studies conducted in support of the present invention and reported in Examples I-III, the effects of lipid acyl chain length and degree of unsaturation, lipid charge, and presence of sterol on in vitro drug efflux rates from liposomes were examined. Of factors which were studied, the most important is acyl chain length and the degree of unsaturation in the phospholipid(s) forming the liposomes. The study reported in Example I shows that the in vitro drug efflux half life (a measure of the time required for one-half of the liposome-entrapped drug to be released from the liposomes on incubation in vitro) can vary over a hundredfold or more, according to acyl chain length and degree of unsaturation. In forty different phosphatidylcholine (PC) lipids and lipid mixtures which were studied, increasing either acyl chain length, or the degree of saturation of phospholipid acyl chains, led to greater drug efflux half lives, with the most significant increases being observed when the liposomes included a significant proportion of lipids whose transition temperature ($T_c$) are above the temperature at which the efflux half lives are measured, e.g., 37°C.

Studies on the effect of lipid charge on drug release rates, reported in Example II, indicate that the addition of a negatively charged lipid, such as phosphatidyl glycerol (PG), at a mole ratio of about 10%, produces a slight to moderate increase in efflux half life. The lipid-charge effect is dependent somewhat on the degree of saturation and chain length in the charged and uncharged lipids used in forming the liposomes, with the charged effect producing a greater increase in drug efflux rate where the liposomes are formed of predominantly shorter and/or unsaturated lipids, and producing less effect in the case of longer-chain and/or saturated lipids.

The effect of sterol, either cholesterol or the negatively charged cholesterol, hemisuccinate, on drug efflux from liposomes is considered in Example III. A first study involved liposomes formed with one of eight PC lipids or lipid mixtures and cholesterol, at a PC/cholesterol mole ratio of 60:40. In general, cholesterol was found to have a moderating effect on the acyl composition of the phospholipid, slightly increasing drug efflux half lives in liposomes containing predominantly unsaturated and shorter-chain length PCs, and producing a significant decrease in the drug efflux half lives in liposomes containing predominantly longer-chain and more saturated phospholipids. This moderating effect may be due to the known effect of cholesterol in decreasing the fluidity of unsaturated membranes (which would produce greater efflux half lives) and increasing the fluidity of saturated membranes (which would produce shorter half lives).

A negatively charged sterol, such as cholesterol hemisuccinate, when formulated with uncharged phospholipid(s), appears to have little effect on drug efflux rates over that observed in liposomes formed from uncharged phospholipids and cholesterol (Example III). Surprisingly, however, the drug efflux half lives observed in liposomes composed of uncharged phospholipids and cholesterol are enhanced significantly by the addition of negatively charged phospholipid (10 mole percent PG). As seen from the data presented in Table 5 of Example III, in nearly all of the PC mixtures which were studied, the addition of 10 mole percent PG to liposomes also containing PC (50 mole percent) and cholesterol (40 mole percent) enhanced the drug efflux half life more than twofold, and in some cases more than threefold over those observed for liposomes composed of PC and cholesterol only. The combination of a neutral phospholipid, such as PC, a charged phospholipid, such as PG, and cholesterol appears particularly advantageous in achieving a range of drug half lives, as measured at 37°C, of between about 2 and 24 hours.

The lipids forming the liposomes may, of course, include phospholipids and sterols other than the model lipids used in the above-mentioned studies, and may also include other types of lipids, such as glycolipids and sphingolipids, which are compatible with liposome formation. A list of lipids which are used commonly in liposome formation is given on page 471 of reference 2. The liposomes may also be formulated to include various types of drug-protective or lipid-protective agents, such as the antoxidant $\alpha$-tocopherol, which was typically included at a 1.0 mole percent in the liposomes described herein.

Another factor in the design of liposomes having a selected drug efflux rate is the interaction of the drug with the liposome bilayer(s). The studies reported in Examples I-III involve a water-soluble drug, metaproterenol sulfate (MPS), which is entrapped in liposomes predominantly in encapsulated form, i.e., in the aqueous vesicular space of the liposomes. For more lipophilic drugs, which tend to partition in the lipid bilayer phase of the liposome, the relative effects of acyl chain lengths and saturation, sterol content, and lipid charge density may produce somewhat different effects on drug efflux rate. The procedures described in Examples I-III for determining the effect of various lipid-composition parameters on drug efflux half lives are recommended for determining lipid-composition effects on efflux of other selected lipid-soluble or water-soluble drugs.

## IB. Liposome Preparation

The liposomes may be prepared by a variety of techniques, such as those detailed in reference 2. The choice of the liposome preparation method will depend, in part, on the nature of the drug to be entrapped. As defined herein, "drug" is intended to include any pharmacologically active agent which has a site of action in the respiratory tract or is therapeutically active when it is taken up systemically from the respiratory tract. Such drugs may include antibiotics, peptide hormones, enzymes, enzyme inhibitors, anti-tumor agents, bronchodilators, allergens, antihistamines, and biogenic compounds such as steroids and prostaglandins.

For purposes of discussion, three general classes of drugs will be considered. The first class are water-soluble, liposome-permeable drugs which are characterized by a tendency to partition preferentially into the aqueous compartments of the liposome suspension, and to equilibrate, over time, between the inner lipsomal spaces and outer bulk phase of the suspension. The time required for equilibration is, of course, related to the drug-release rate of the liposomes, and will therefore vary according to the lipid composition of the liposomes. As seen above, drug-release half lives of between a few hours and days are typical. Also, each drug will have its own characteristic release rate, depending on drug size, charge, and ability to interact with the liposome membrane. Representative drugs in this class include terbutaline, albuterol, atropine methyl nitrate, cromolyn sodium, propranalol, flunoisolide, ibuprofen, gentamycin, tobermycin, pentamidine, penicillin, theophylline, bleomycin, etoposide, captopril, n-acetyl cysteine, and verapamil.

A second general class of drugs are those which are water-soluble, but liposome-impermeable. For the most part, these are peptide or protein molecules, such as peptide hormones, enzymes, enzyme inhibitors, apolipoproteins, and higher molecular weight carbohydrates are characterized by long-term stability of encapsulation. Representative compounds in this class include calcitonin, atriopeptin, $\alpha$-1 antitrypsin (protease inhibitor), interferon, oxytocin, vasopressin, insulin, interleukin-2, superoxide dismutase, tissue plasminogen activator (TPA), plasma factor 8, epidermal growth factor, tumor necrosis factor, lung surfactant protein, and lipocortin.

In the third class of drugs are lipophilic molecules which tend to partition into the lipid bilayer phase of the liposomes, and which are therefore associated with the liposomes predominantly in a membrane-entrapped form. The drugs in this class are defined by an oil/water partition coefficient, as measured in a standard oil/water mixture such as octanol/water, of greater than 1 and preferably greater than about 5. Representative drugs include prostaglandins, amphotericin B, progesterone, isosorbide dinitrate, testosterone, nitroglycerin, estradiol, doxorubicin, beclomethasone and esters, vitamin E, cortisone, dexamethasone and esters, DPPC/DPPG phophoslipids, and betamethasone valerate.

One preferred method for preparing drug-containing liposomes is the reverse phase evaporation method described in reference 3 and in U.S. Patent No. 4,235,871. In this method, a solution of liposome-forming lipids is mixed with a smaller volume of an aqueous medium, and the mixture is dispersed to form a water-in-oil emulsion. The drug to be entrapped is added either to the lipid solution or aqueous medium. After removing the lipid solvent by evaporation, the resulting gel is converted to liposomes, with an encapsulation efficiency, for a water-soluble drug, of up to 50%. The reverse phase evaporation vesicles (REVs) have typical average sizes between about 2-4 $\mu$m (microns) and are predominantly oligolamellar, that is, contain one or a few lipid bilayer shells. The oligolamellar nature of the vesicles may facilitate drug efflux and thus contribute to a lower efflux half live for an encapsulated drug.

A simple lipid-hydration procedure for producing multilamellar vesicles (MLVs) may be preferred where high drug encapsulation efficiency is not desired. In this procedure, a mixture of liposome-forming lipids dissolved in a suitable solvent is evaporated in a vessel to form a thin film, which is then covered by an aqueous solution of the drug. The lipid film hydrates to form MLVs, typically with sizes between about 0.1 to 10 $\mu$m (microns). As in the REV method, the drug to be encapsulated is added either to the initial lipids or the the hydrating medium, depending on its solubility in water. The percent of total drug material which can be encapsulated in the MLVs, calculated as the ratio of encapsulated drug to total drug used in vesicle preparation, is typically between about 5-20% for water-soluble drugs. As seen in Examples I-III, encapsulation efficiency is dependent to a degree on lipid composition.

Following liposome preparation, the dispersion can be treated by extrusion to reduce overall liposome size and size heterogeneity. Reducing liposome particle size may be important in achieving efficient aerosolization of the liposomes, and in maximizing drug deposition in a desired portion of the repiratory tract. Experiments conducted in support of the present invention indicate that liposomes contained in an aqueous suspension may be aerosolized efficiently in the form of liposome-containing aqueous particle mist, where the liposomes are predominantly less than about 1 micron in diameter. In terms of targeting the liposomes to a particular region of the respiratory tract, it is advantageous to produce liposomes whose sizes are compatible with a desired size range of mist particles. For example, mist particle sizes of greater than about 5 microns would favor deposition in the upper respiratory-tract regions, and particle sizes of between about 0.5-1.5 $\mu$m (microns) would favor deposition in lower pulmonary regions of the respiratory tract. Generally liposomes having particle sizes less than about 1 micron are compatible with a wide range of mist particle sizes, e.g., from 1-10 $\mu$m (microns). As will be seen below, where the liposomes are aerosolized in the form of an aqueous mist, the actual sizes of particles carrying the liposomes may be much greater than that of the liposomes themselves, and thus actual liposome size becomes less crucial to targeting.

One effective sizing method involves extruding an aqueous suspension of the liposomes through a polycarbonate membrane having a selected uniform pore size, typically 0.2, 0.4, 0.6, 0.8 or 1 $\mu$m (microns) (reference 3). The pore size of the membrane corresponds roughly to the largest sizes of liposomes produced by extrusion through that membrane, particularly where the preparation is extruded two or more times through the same membrane. This method of liposome sizing is used in preparing the liposomes described in the several examples below. A more recent method involves extrusion through an asymetric ceramic filter. The method is detailed in co-owned U.S. patent for Liposome Extrusion Method, US-A-4,737,323, filed February 13, 1986.

The liposomes may be further treated to remove unencapsulated drugs and/or other solute material. Conventional separation techniques, such as centrifugation, diafiltration, and molecular-sieve chromatography are suitable. The separation step should be carried out after sizing, since the sizing procedure itself can lead to liposome rupture and release of encapsulated drug. It will be appreciated that free drug removal will be necessary to remove water-soluble, liposome-impermeable drugs from the liposome dispersion. In the case of a lipophilic or liposome-permeable drug, the relative percent of free drug in a liposome suspension can be reduced by concentrating the liposomes, according to methods described in Section IC.

IC. Liposome Suspension: Impermeable Water-Soluble Drugs

According to an important feature of the invention, the liposome suspension which is administered to respiratory tract contains the therapeutic drug of interest predominantly in a liposome-entrapped form. By this is meant that at least about 50%, and preferably at least about 70% of the drug, is contained in a liposome-associated form, i.e., either encapsulated or membrane-entrapped form. For water-soluble, liposome-impermeable drugs, the suspension may be a dilute liposome dispersion in which only about 5%-30% of the total aqueous volume is encapsulated within the liposomes.

A dilute suspension of liposomes is readily prepared, as above, by forming liposomes in the presence of the drug, sizing if desired, then removing free drug from the suspension. The final concentration of encapsulated drug should preferably be such that the liposomes aqueous spaces are iso- or hypo-osmotic with respect to the environment of the respiratory tract, to prevent liposome damage by swelling at the delivery site. The final concentration of liposomes in the suspension is adjusted to provide a selected quantity of liposome-encapsulated drug in a given volume of aerosolized spray. For example, where a maximum amount of drug is to be delivered in a given suspension volume, the concentration of encapsulated drug should be close to isotonicity, and the suspension concentrated to between about 20%-30% encapsulated aqueous volume, i.e., the highest liposome concentration which allows direct aerosolization.

ID. Liposome Suspension: Permeable Water-Soluble Drugs

Where the water-soluble drug is also freely permeable across the liposomal barrier, it is necessary to prepare the suspension in a form in which the drug is confined predominantly to the liposomes, after complete equilibration of the drug between the inner liposomal and outer bulk phase compartments of the suspension. This condition is achieved, according to the one embodiment of the invention, in an aqueous liposome paste in which the encapsulated volume of aqueous medium is greater than about 50% and preferably about 70-75% of the total suspension volume, reflecting the approximate percentage of liposome encapsulated drug.

In a preferred method of forming the paste, a relatively dilute liposome dispersion, prepared as above, is concentrated by ultrafiltration, and more preferably, tangential-flow ultrafiltration. An multi-filter apparatus for carrying out the filtration process has been described in co-owned patent application for "Liposome Concentrate and Method", filed May 7, 1986. Briefly, the dispersion is recyled continuously through a frame-and-plate type filtration device having one or more filtration units, each composed of a central filtrate screen and a pair a filters supported on the upper and lower sides of screen. The dispersion is pumped under pressure across both membranes, and aqueous medium is drawn though the filters across a pressure gradient of typically about 34.5 to 172 kPa (5-25 psi), with higher gradients being required as the recycled material becomes more concentrated. The material is recylcled through the system until a desired liposome concentration, which can be as high as 70-75% liposomes, expressed in percentage of liposome-encapsulated volume, is achieved. The ultrafiltration membranes used in the device may be conventional membranes, such as polysulfone, nylon, or cellulose acetate membranes which have a size cutoff of between about 10,000-100,000 daltons.

Other concentration methods, such as drying and centrifugation to pellet liposomes, may also be used to form the liposome concentrate of the invention, although ultrafiltration is preferred, because of advantages of sterility, large-volume capacity, and relatively mild conditions to which the liposomes are exposed.

In another general embodiment, the liposome paste from above is emulsified in a propellant solvent, to form a double emulsion of liposomes suspended in concentrated form within aqueous emulsion particles which are themselves suspended in the propellant solvent.

The propellant solvent is one in which (a) liposomes can be suspended without damaging the lipid integrity of the particles, and (b) the drug has a relatively low partition coefficient with respect to water, such that the bulk of the drug remains associated with the liposomes. Example V below examines the stability of liposome emulsions in six fluorocarbon propellants. The propellants which were tested are "Freon 11" ($CCl_3F$), "Freon 12" ($CCl_2F_2$), "Freon 22" ($CHClF_2$), "Freon 113" ($CCl_2FCClF_2$), "Freon 114" ($CClF_2CClF_2$), and "Freon 115" ($CClF_2CF_3$). Three different formulations of liposomes were tested, and all showed good stability in Freon 113, 114, and 115. Best stability was achieved with liposomes containing unsaturated acyl-chain phospholipids . It is noted here that Freon 114 and 115 can be mixed in proportions which give a proper vapor pressure at room temperature for an aerosol canister. Dimethylether, another well-known propellant solvent, may be added to a fluorocarbon propellant to decrease solvent density, but the amount added must be carefully monitored to prevent solvent damage to the liposomes.

To form the double emusion, the liposome paste from above is added to the propellant solvent, at a final volume concentration of between about 5%-30% preferably. The mixture may further contain an emulsifier, such as Tween-80, to enhance the stability of the emulsion. Vigorous mixing, for example, by sonication, is used to produce the final emulsion.

In another embodiment, the liposomes suspension is prepared by dispersing dried liposomes, in powdered or particulate form, in a propellant solvent of the type just considered. The dried liposomes preferably include a bulking agent, which is added to improve the formulation in several ways: The agent improves the milling properties of the dried material, and also increase the density of the particles, to give better dispersion in relatively dense fluorocarbon propellants. The bulking agent also acts to shield the liposomes and the encapsulated drug from the propellant and provides greater particle cohesion in the suspended form. There is also evidence that the bulking agent reduces membrane damage on drying and dehydrating, so that less free drug is produced when the dried liposomes rehydrate in the moist environment of the lung. Finally, since the hydroscopic nature of the bulking agent improves hydration during passage through the airways. Preferred stabilizing agents include a variety of mono- or disaccharides, such as glucose, mannitol, trehalose, sucrose, and lactose, which are tolerated well in the respiratory tract.

Methods for forming liposomes containing such bulking agents in encapsulated form are described in co-owned patent for "Stabilized Liposome/Amphotericin Composition and Method, US-A-4,766,046, filed October 17, 1985. In one preparation method, a solution of vesicle-forming lipids are added in an organic solvent to bulking agent particles, and the lipid solvent is evaporated, depositing the lipids as a coating on the particles. The preparation method now follows the above MLV procedures, with addition of hydrating medium producing MLVs with dissolved bulking agent contained in the encapsulated and outer bulk phase aqueous compartments. Non-encapsulated agent may be removed before dehydrating the liposomes.

The liposome dispersion or paste is dehydrated by known techniques, which may include lyophilization, or spray drying under vaccuum conditions. After drying, the liposomes are particlized by milling or the like, to a final size of less than about 5 $\mu$m (microns), and preferably, less than about 1 $\mu$m (microns). The liposome powder is added to the propellant solvent to a final concentration preferably between about 1-10% by weight.

### D. Liposome Suspension: Lipid-Soluble Drugs

The liposome suspension for use with lipophilic drugs is an aqueous suspension whose lipid concentration is at least sufficient to ensure that 50% or more and preferably 70% or more of the drug is in the lipid (liposome bilayer) phase of the suspension, and within the liposomes. The liposome concentration required will depend on the partition coefficient of the drug, as well as other factors mentioned above. If the drug partition constant is only slightly greater than 1, the suspension would have to be concentrated to a paste-like consistency in which about half or more of the total aqueous volume is liposome encapsulated. With a partition coefficient of about 10, a much more dilute aqueous dispersion would be possible, Of course, the extent of allowed dilution is also limited by the need to entrap an adequate drug dose in a fairly small amount of aerosolized material.

### II. Aerosolizing Liposomes

The present inhalation system also includes a device for aerosolizing the liposome suspension in a form suitable for inhalation. These devices are discussed below in relation to the three general types of lipsome suspensions to be nebulized.

### IIA. Dilute Liposome Suspensions

Pneumatic nebulizers which are designed for atomizing aqueous sample are commercially available, and operate according to well-known procedures, such as those outlined in Example IV. Typically, the nebulizing operation is carried out at a pressure of about 34.5-103 kPa (5-15 psi), and the aqueous particles formed are typically in the range of about 2-6 $\mu$m (microns). The device may be controlled to produce a measured quantity of aerosolized liposomes, according to known operational variables (reference 4).

Because the aerosolization procedure may result in mechanical disruption of a liposome suspension, it may be important to establish that the nebulizing process does not significantly affect liposome integrity and size. The effect of aerosolization by a pneumatic nebulizer on liposome particle size and loss of encapsulated material is reported in Example IV. Here four liposome compositions having in vitro drug efflux half lives ranging from 206 minutes to 5256 minutes, and each having an original particle size distribution between about 0.180-0.300 $\mu$m (microns), were examined for size change and loss of encapsulated material after aerosolization at 34.5 kPa (5 psi). As reported in Table 7 of Example IV, no change in liposome size or loss of encapsulated material was observed for three of the preparations, and only a slight loss of encapsulated material and moderate increase in size was observed for liposomes with the lowest drug efflux half life.

Another device suitable For aerosolizing an aqueous suspension of liposomes, and preferably a relatively dilute suspension containing less than about 25%-30% encapsulated aqueous volume, uses ultrasonic energy to break up a carrier fluid into a fine mist of aqueous particles. The ultrasonic nebulizer device has been found to produce a liposome aerosol mist whose particle sizes are about the same as those formed by a compressed air nebulizer, i.e., between about 2-6 $\mu$m (microns). The effect on liposome size and retention of encapsulated material by ultrasonic aerosolization was examined in a study similar to the one just described, and also reported in Example IV below. The results of that study, summarized in Table 8 in Example IV, are consistent with the earlier study, showing that the aerosolizing process has substantially no effect on liposome size or release of encapsulated material, except a relatively minor effect on the liposomes composed predominantly of short, unsaturated acyl chain lipids.

## IIB. Liposome Paste Suspensions

For aerosolizing a liposome paste of the type used for delivery of a water-soluble, liposome-permeable drug, the paste is first mixed with a carrier solvent, to form a diluted dispersion which can be aerosolized. The carrier solvent may be an aqueous medium, in which case the paste is diluted to a form suitable for spraying, such as by a pneumatic or ultrasonic nebulizer. The amount of diluent added is sufficient to dilute the paste to a sprayable dispersion, preferably containing less than about 30% total encapsulated volume. Assuming the paste has an initial encapsulated volume of 70-75% of the total paste volume, it can be appreciated that a given volume of the paste must be diluted with at least one and two volumes of diluent.

Alternatively, the carrier solvent may be a propellant solvent, which when mixed with the liposome paste, forms either a paste-in-propellant emulsion, of the type described above in Section ID, or a foamed paste mixture formed by introducing pressurized propellant into the paste. The volume ratio of paste to propellant is preferably between about 10%-30%, giving an emulsion or foam composition which can be readily aerosolized.

A suitable aerosolizing device for the making and spraying the emulsion includes (1) separate chambers for holding the paste and carrier vehicle liquids, (2) a mixing chamber where metered amounts of the two liquids are mixed, and (3) means for aerosolizing the contents of the mixing chamber. Dispensing devices designed for storing two different liquids in isolation from each other, and for intermittant dispensing of the liquids in contact with each other are known, as disclosed, for example, in U.S. patent # 3,325,056. Where the device operates to mix the paste with an aqueous carrier medium, the pneumatic or ultrasonic energy used to produce aerosolization can also be used to effect rapid and complete mixing of the two fluids.. The releasing means includes valve structure communicating with the mixing chamber, to allow release of the contents of the mixing chamber through a nozzle. Here it is noted that the mixing and spraying steps are carried out in relatively quick succession, before appreciable leakage of encapsulated drug into the aqueous carrier vehicle can occur, i.e., while the drug is still predominantly in liposome-encapsulated form.

In the case of a self-contained nebulizer containing a propellant carrier fluid, the two fluids may be mixed under conditions which cause rapid breakup of the paste by gassification or foaming, just prior to ejecting the gassified mixture through a nozzle to achieve aerosolization. Alternatively, the device can operated to entrain the paste in emulsion form in the liquid propellant, just prior to ejecting the mixture. The releasing means is a valve structure which communicates with the mixing chamber, to allow release of the self-pressurized contents of the chamber through a nozzle. The propellant solvent is preferably a fluorocarbon or fluuorocarbon/diethylether mixture, such as discussed above, which does not itself cause appreciable liposome disruption. However, since the liposomes are in contact with solvent for only a brief mixing and spraying period, problems related to liposome disruption and drug partitioning across the liposomal barrier are less serious than where the bulk phase of the liposome suspension is itself a propellant solvent, i.e., where the liposomes are initially formulated in a propellant solvent.

## IIC. Preformed Liposome/Propellant Suspensions

Valved container devices for aerosolizing particulate material suspended in a propellant solvent are well known. These devices generally operate by supplying a particle suspension to a metering chamber, and forcing the contents of the chamber through a nozzle under pressure, to aerosolize a metered quantity of the suspension. In the present invention, the particle/propellant suspension is either a suspension of dried particulate liposomes, or a suspension of liposome paste emulsion particles, prepared as in Section ID above. With both types of emulsions, particle separation from the propellant on standing may require that the container be shaken vigorously before use, to resuspend the liposomes uniformly in the solvent. It is noted that almost all self-contained aerosol devices require vigorous shaking before use.

## III. Therapeutic Applications

A variety of respiratory tract conditions and diseases, including nasal congestion, asthma and other bronchospasm conditions, nasal inflammation, and vascular headaches, may be treated by drug inhalation therapy according to the present invention. Representative drugs which are useful in the treatment of respiratory tract conditions and diseases are listed in reference 1, and include nasal decongestants such a propylhexedrine and methylhexylamine; respiratory stimulants such as epinephrine, isoproteranol and metaproterenol, and isoetharine; corticosteroids such as dexamethasone and beclomethasone; and other medications such as cromolyn, sodium acetyl cysteine, and ergotamine tartrate.

In preparing liposomes for targeting a drug to the respiratory tract, the liposomes are formulated to produce a selected in vitro drug efflux rate, preferably measured at about 37°C, for achieving a controlled drug release at the target site in the respiratory tract. It has been seen in Section IA how lipid-composition factors can be varied to achieve in vitro drug half lives ranging from as short as half an hour or less up to several days. According to another important aspect of the invention, the liposome composition can be varied selectively to moderate unwanted systemic side effects observed when the drug is taken up systemically from its site of deposition in the pulmonary region of the respiratory tract. Generally, the same lipid composition factors which lead to longer drug efflux half lives are effective in reducing systemic drug levels which are observed shortly after administration of the drug by inhalation. For example, to produce greater reduction in initial systemic drug level effects, the liposomes may be formulated with a greater percentage of long-chain and/or saturated acyl chain phospholipids.

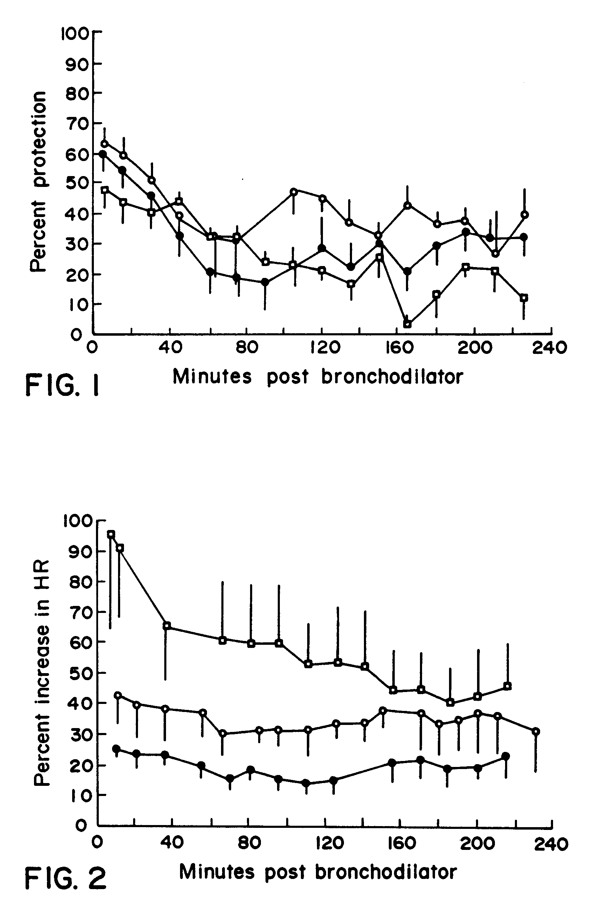

The application of the method and system of the invention for the treatment of bronchoconstriction is illustrated in Example VI below. The animals studied were treated by inhalation with MPS in free form or encapsulated in one of two different-composition liposomes--one having an in vitro drug efflux half life of 250 minutes and the other, an efflux half life of about 1150 minutes. Both liposome formulations were delivered in aqueous aerosol particles in which the drug was contained predominantly in encapsulated form, and the amount of MPS delivered was about the same for both liposome-encapsulated and free MPS. After drug administration, the degree of protection against bronchoconstriction provoked by aerosols of acetylcholine solution was measured approximately every 15 minutes for a four hour period. Typical results for two animals, representing the average of three runs for both free drug and the two liposome-encapsulated drug formulations, are shown in Figure 1. The average percent protection values are indicated by open squares for the free drug, by closed circles for the long half-life (1150 minutes) liposome formulation, and by open circles for the short half-life (250 minutes) liposome formulation. As seen from the data, both free and liposome-encapsulated drug provided about the same degree of protection as the free drug.

The above MPS inhalation treatment also illustrates the ability of liposome-encapsulated MPS to protect the animal against tachycardia, a relatively severe systemic side effect seen when MPS is administered by inhalation in free form. The effect of free drug and liposome-encapsulated drug on percent increase in heart rate during the four hour period after MPS administration by inhalation was also measured. Typical results, representing the average of three experiments for each of free drug and the two liposome-encapsulated drug formulations, are shown in Figure 2. As in Figure 1, the data corresponding to free drug is indicated by open squares, the long half-life liposome formulation, by closed circles, and the short half-life liposomes by open circles. As seen from the figure, the free drug produced an approximately 90% increase in heart rate within about 5 minutes of drug administration, and this heart rate gradually subsided over the four hour observation period to about a 50% level.

By contrast, treatment with the same concentration of drug, but contained in liposome formulation, produced an initial increase in heart rate observed in the first half hour after drug administration, of between 20%-30%. This rate remained substantially constant over the four hour period, evidencing the fact that a relatively small amount of drug is delivered in utilizable form initially, but is delivered to the site of absorption at a relatively constant rate over an extended drug-delivery period.

The short half-life liposome formulation (open circles in Figure 2) also protected against high initial heart rate increase and showed substantially constant systemic drug levels over the four-hour monitoring period. Interestingly, the measured heart rate for the short half-life formulation was uniformly about two times higher than that of the long half-life formulation throughout the monitoring period.

A similar group of tests were performed to measure the therapeutic and systemic side effects produced by delivering liposome-encapsulated MPS to anesthetized guinea pigs. The studies involved the four liposomes compositions: (1) EPG/EPG/cholesterol/α-T (5:1:1:0.1), which has a MPS release half life of 250 minutes (Example III); (2) DSPC/DSPG/cholesterol/α-T (5:1:1:0.1), which has a MPS release half life of 1148 minutes (Example III); (3) DPPC/DPPG/cholesterol/α-T (5:1:1:0.1), which has a MPS release half life of 941 minutes (Example III); and (4) DPPC/DPPG/α-T (9:1:0.1), which has a MPS release half life of 5256 minutes (Example II). Each composition contained 1% MPS, predominantly in encapsulated form.

The animals were administered one of the four liposome compositions, or saline (as a control) or free drug. The animals in each test group were stabilized for 15 minutes after anesthesia, then administered a controlled volume of aerosol by intubation for a two minute period. At, 15, 45, 90, and 105 minutes after aerosol delivery, the animals recived a dose of histamine, over a fifteen second interval, to cause transient, asthma-like bronchoconstriction. The animals were monitored for airway resistance, dynamic compliance, heart rate, and blood plasma levels of MPS at 30 minute intervals over a two hour period after aerosol administration. The airway resistance and dynamic compliance measurements indicate the degree of protection provided by the MPS treatment against the histamine-induced bronchoconstriction. Formulation 1, with a relatively high release rate (low drug release half life) was about as effective as free drug in preventing increased airway resistance and dynamic compliance, whereas formulations 2 and 3, both with intermediate drug-release rates, showed therapeutic effects which were intermediate between free drug and saline control. Interestingly, formulation 4, which has the lowest drug release rate in vitro, was similar to formulation 1 and free drug in its protection against airway resistance and dynamic compliance increases. The explanation for this anomaly may be that the DPPC/DPPG lipids in the formulation 4 liposomes are dominant phopholipids in the respiratory tract, and may therefore exchange phospholipids with or become integrated into the lipid membranes at the target site relatively rapidly, with accompanying release of drugs. This supposition is supported by data on systemic drug effect, discussed below, which indicates that formulation 4 liposomes give systemic drug levels which are intermediate between those of formulation 1 and and those of formulations 2 and 3. It is also possible that the DPPG and DPPG lipids in the formulation 4 liposomes are therapeutically effective, independent of the release of MPS.

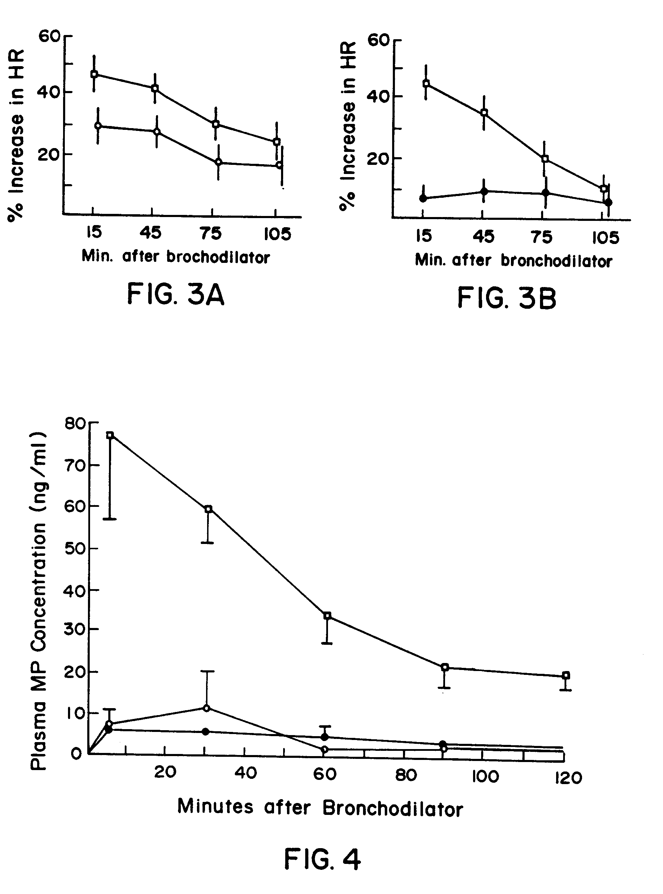

The percent increase in heart rate provides, as in the earlier study, a measure of the systemic action of the drug. As seen in Figures 3A and 3B, free MPS (open squares in the figures), produces a large initial increase in heart rate, which then falls slowly over the next 90 minutes to a level about 20% above normal. This percent increase is much smaller when the drug is administered in encapsulated form, either in formulation 1 liposomes (open circles in Figure 3A), or in formulation 4 liposomes (closed circles in Figure 3B). Also consistent with the earlier study, the more stable liposome formulation (4) was substantially more effective in reducing the unwanted heart stimulation side effect than the formulation 1 liposomes. Although not shown in the figures, the two intermediate formulations 2 and 3 gave heart rate increases, reflecting systemic drug levels, which were both appreciably lower than seen with formulation 4.

The effect of liposomes in reducing MPS levels in the bloodstream after drug delivery to the lungs is more dramtically apparent from the plasma MPS concentration levels shown in Figure 4. With free drug, plasma drug levels were nearly 80 ng/ml initially, with a slow decline over a two-hour period to about 20 ng/ml. By contrast, with all of the liposome formulations, plasma drug levels were maintained at a susbstantially constant level below about 10 ng/ml throughout the test period.

The above data demonstrate two important features of the invention. The first is the ability of liposomes to moderate the initial and extended drug-level effects of a drug administered by inhalation. That is, the liposomes function to "flatten" the drug-release curve seen for free drug by reducing initial drug-level effects and maintaining elevated drug levels over an extended period. The second feature is the ability to control or regulate drug release rates in vivo using liposomes having a suitably selected in vitro drug efflux rate. As seen in the above system, the selected rate of drug release can be varied up to about two fold by suitable selection of liposomes whose in vitro drug efflux rates vary over about a 4-5 fold range.

In formulating liposomes for use in inhalation treatment, it is of course necessary to achieve a therapeutically effective dose of drug during the period of sustained drug release, and for this reason, liposomes having very long drug efflux half lives may be suboptimal therapeutically. In the bronchoconstriction treatment described above, the comparable therapeutic effect of liposome-encapsulated MPS, with the respect to free drug, suggests that even the long half-life liposomes are functioning to release the drug at a concentration which is optimal or near-optimal for therapeutic effect.

According to an important aspect of the invention, the inhalation system and method just described are advantageous for delivering drugs systemically, as well as for local respiratory tract delivery. The controlled drug-release rates for a sytemic-acting water-soluble drug (MPS) are seen above. Lipid soluble drugs, which are contained predominantly in the lipid bilayer region of liposomes, gradually become associated with endogenous lung lipids, with liposome deposition in the lower respiratory tract, and in this form, the drugs can traverse the blood-gas barrier to enter the pulmonary circulation via concentration-dependent diffusion.

Delivery of drugs in a predominantly liposome-associate form solves a variety of problems encountered when the drug is administered in free or predomianantly free form. In addition to the drug moderating and sustained-release features seen above, the liposomes act to protect the drug from oxidation and protect the respiratory tract from potentially irritating drugs, particularly those which, because of solubility properties, must be administered in micronized form.

In one application for intrapulmonary drug delivery, $\alpha$-1-protease inhibitor is delivered to the pulmonary interstitium in liposome-encapsulated form to stem the development of pulmonary emphysema. The liposomes act to protect the protease inhibitor's tertiary structure from oxidation, and facilitate its transport across the pulmonary cell membranes.

An example of a specific application for systemic drug delivery is the encapsulation and delivery of nitroglycerin, a coronary vessel dilator used to relieve the symptoms of angina pectoris. The drug formulation typically will contain about 30%-40% free drug, which can be rapidly absorbed by the pulmonary blood flow and transported directly to the heart, its primary site of action, to provide immediate relief from the chest pain associated with angina. The remaining 60%-70% or more of the liposome-encapsulated drug is released slowly, at a rate controlled by liposome composition, to afford prolonged coronary vessel dilation, and thus relief from chest pain for an extended period.

Oxytocin, a peptide hormone that induces and augments the strength of uterine muscle contractions during labor, can be formulated and delivered in a manner similar to that described for nitroglycerine. It is currently delivered by intravenous infusion, a process that requires placement and maintenance of a venous cannula, a sometimes difficult procedure that limits the patient movement and posture. Aerosols of liposome-oxytocin formulation would provide immediate and sustained delivery to the systemic circulation, similar to that provided by IV infusion, without restricting patient motion.

From the foregoing, it can be appreciated how various objects and features of the invention are met. The method of the invention allows for administration of lipid- or water-soluble drugs, under conditions which produce a controlled release of drug at the target site. The controlled release of drug may serve the purpose of extending the time interval between drug administrations, moderating short and long-term cyclical variation in drug levels, either at the site of deposition or systemic drug levels, and/or substantially reducing unwanted systemic side effects.

The liposomes used in the method and system of the invention can be formulated to achieve a desired controlled drug release rate, by formulating the liposomes to produce a selected in vitro drug efflux rate.

The inhalation method and system are compatible with a variety of different aerosolization procedures which allow for the administration by inhalation of liposomes in various sized aerosol particles and form, ranging from rather large aqueous particles to relatively small powdered liposome preparations.

Toxicity studies conducted in support of the invention showed no adverse effects of liposomes administered by inhalation. Under the conditions of the study acute (four hour) exposure to a single dose of aerosolized liposomes), there were no statistically significant differences between a phosphate-buffered saline (blank) and two liposome-exposed mouse populations in body weights, fresh excised lung weights, and light-microscope histopathologic findings.

The following examples are intended to illustrate various features and uses of the present invention. Trade names and registered trade marks are acknowledged as such.

Example I

Effect of Lipid Chain Length and Saturation on Drug Efflux

Metaproteranol sulfate (MPS) was obtained from Vinchem Inc. (Chatham, NJ); $\alpha$-tocopherol ($\alpha$-T), from Sigma Chemical Co. (St. Louis, MO); and $^{14}$-C sucrose, from Amersham Co. (Arlington Hts, IL). Egg phosphatidyl choline (EPC), soy phosphatidyl choline (SPC), hydrogenated egg phosphatidyl choline (HEPC), hydrogenated soy phosphatidyl choline (HSPC), dioleoyl phosphatidyl choline (DOPC), dimyristoyl phosphatidyl choline (DMPC), dipalmitoyl phosphatidyl choline (DPPC), and distearoyl phosphatidyl choline (DSPC), were obtained from Avanti Polar Lipids (Birmingham, AL). The 8 PC lipids have the transition temperatures ($T_c$) and fatty acyl chain compositions given in Table 1 below, where the lipids having greater acyl chain lengths and/or degrees of saturation are arranged in descending order.

Table 1

| PC | $T_c$(C°) | Fatty Acyl Composition |
|---|---|---|
| SPC | -15 to -7 | 16:0(12%) 18:2(73%) |
| DOPC | -22 | 18:1 |
| EPC | -15 to -7 | 16:0(42%) 18:1(28%) 18:2(16%) |
| DMPC | 23 | 14:0 |
| DPPC | 41 | 16:0 |
| HEPC | 55 to 65 | 16:0(36%) 18:0(60%) |
| HSPC | 55 to 65 | 16:0(13%) 18:0(87%) |
| DSPC | 55 | 18:0 |

For each of the 8 PC lipids, multilamellar vesicles (MLVs) were prepared by dissolving 350 $\mu$mole of the selected PC lipid with 1 mole percent of $\alpha$-T in chloroform. The dissolved lipid was evaporated to dryness under vacuum in a round-bottom flask to form a lipid film on the flask walls. A 5 ml solution containing 20 mg/ml MPS and 1.1 x $10^7$ cpm $^{14}$C-sucrose in phosphate-buffered saline (PBS), pH 7.2, 290 mOsm, was added to the flask to cover the film. At the end of a 2-hour hydration period, the liposomes (MLVs) which formed were extruded successively two times each through a 0.4 and 0.2 $\mu$m (micron) uniform pore-size polycarbonate filter (Bio-Rad; Richmond, CA). Unencapsulated MPS and sucrose were removed by passage of the extruded MLVs through a Sephadex G-75 gel-exclusion column. The percent encapsulation of MPS ranged from a low of about 0% for DMPC MLVs to a high of 21% for DPPC MLVs (Table 2). Encapsulation of $^{14}$C-sucrose ranged from a low of 0.2% for DMPC MLVs to a high of 18% for the DPPC MLVs.

To measure leakage of encapsulated solutes from the MLV preparations, each preparation was incubated at 37°C for a period of up to 42 hours. Timed aliquots taken periodically during the 42-hour incubation period were passed through a gel-exclusion column, and the lipid, MPS, and sucrose present in the liposome peak were assayed by conventional methods. The loss of lipid-associated drug during the course of the incubation was used to determine the drug efflux half life ($t_{1/2}$) for each preparation, calculated as the time required for the drug/lipid ratio to fall by one-half. The calculated efflux half lives, expressed in minutes, are shown in Table 2 below.

Table 2

| Encapsulation | | |
|---|---|---|
| PC/$\alpha$-T (10:0.1) | % MPS Encapsulated | $t_{1/2}$ MPS (minutes) |
| SPC | 6 | 22 |
| DOPC | 4 | 41 |
| EPC | 10 | 48 |
| DMPC | 0 | -- |
| DPPC | 21 | 574 |
| HEPC | 5 | 6426 |
| HSPC | 8 | 2175 |
| DSPC | 13 | 6366 |

As seen from data in Table 2, the rate of efflux of drugs from the MLVs can be selectively increased from a half life of about 22 minutes to one showing no appreciable leakage over a 42-hour period, by increasing the length and degree of saturation of the acyl chains in the phospholipids making up the liposomes. A comparison of the half-life data in Table 2 with the transition temperature data in Table 1 shows that each of the MLV preparations having a relatively high efflux half life (greater than 500 minutes) is composed of a PC whose transition temperature is above the temperature at which the liposomes were incubated. No appreciable loss of the relatively impermeable $^{14}$C-sucrose from the liposomes was detected over the 42 hour incubation period, indicating that the observed drug efflux is not due to liposome breakdown.

Example II

Effect of Charged Lipids on Drug Efflux

Egg phosphatidyl glycerol (EPG), dioleoyl phosphatidyl glycerol (DOPC), and distearoyl phosphatidyl choline (DSPC) were obtained from Avanti Polar Lipids (Birmingham, AL).

Liposomes (MLVs) containing the lipid composition indicated at the left in Table 3 below, were prepared substantially as described in Example I, starting with initial concentrations of 312 $\mu$mole of the selected PC, 35 $\mu$mole of the selected PG, and 3.5 $\mu$mole $\alpha$-T. For each lipid composition, the dried film was hydrated in 5 ml of the MPS/sucrose solution described in Example I, and the liposomes were sized by extrusion through polycarbonate filters and separated from non-encapsulated material by molecular-sieve chromatography, as above. The percentages of encapsulated MPS are shown in the middle column in Table 3. A comparison of the encapsulation data from Tables 2 and 3 shows that the presence of 10% PG generally increased drug encapsulation efficiencies. This effect was also observed for $^{14}$C-sucrose encapsulation.

The eight MLV fractions were each incubated at 37°C for 42 hours, and the drug efflux half lives determined from the measured loss of drug at several intervals over the 42-hour incubation period. The calculated efflux rate half lives are shown at the right in Table 3.

13

Table 3

| PC/PG/$\alpha$-T (9:1:0.1) | % MPS Encapsulated | $t_{1/2}$ (minutes) |
|---|---|---|
| SPC/EPG | 14 | 92 |
| DOPC/DOPG | 13 | 289 |
| EPC/EPG | 7 | 151 |
| DPPC/EPG | 16 | 110 |
| DPPC/DPPG | 23 | 5256 |
| HEPC/EPG | 15 | 526 |
| HSPC/EPG | 14 | 856 |
| DSPC/DSPG | 20 | 14100 |

The addition of 10 mole percent PG to the liposome/lipid mixture had one of two effects relative to PCs alone (Example I). For the shorter, more unsaturated PC acyl chains, an increase in half life for MPS leakage was seen as a result of the negative charge imparted to the bilayer by PG. With the saturated, longer chain length lipids, the more important factor seems to be the degree of acyl chain unsaturation of the added PG--for example, in the case of EPG, decreasing the overall membrane saturation and thus decreasing measured half lives for MPS leakage. Where a PG derivative of the same saturation chain length as the PC was used (DPPG/DSPG), similar or longer half-lives than with PC alone resulted. As in Example I, no appreciable loss of $^{14}$C-sucrose was observed in any of the preparations.

## Example III

### Effect of Cholesterol on Drug Efflux

Eight liposome MLV preparations containing one of the eight PCs shown at the left in Table 4, cholesterol (CH), and $\alpha$-T in a molar ratio of 6:4:0.1, and containing encapsulated MPS and $^{14}$C-labeled sucrose, were prepared as in Example I. The MLV preparations, after extrusion through 0.4 and 0.2 polycarbonate membranes and treatment with molecular-sieve chromatography to remove unencapsulated material, gave the percent encapsulation of MPS indicated in the middle column in Table 4.

The eight MLV fractions were each incubated for a 42-hour period to determine drug efflux half lives in accordance with the methods described above. The calculated half lives are shown at the right in Table 4.

Table 4

| PC/CH/$\alpha$-T (6:4:0.1) | % Encapsulated MPS | $t_{1/2}$ (minutes) |
|---|---|---|
| SPC/CH | 8 | 103 |
| DOPC/CH | 9 | 313 |
| EPC/CH | 10 | 206 |
| DMPC/CH | 9 | 201 |
| DPPC/CH | 11 | 146 |
| HEPC/CH | 11 | 220 |
| HSPC/CH | 10 | 330 |
| DSPC/CH | 13 | 421 |

In the presence of 40 mole percent cholesterol, drug efflux half lives associated with unsaturated, shorter-chain lipids were longer than those obtained for PC alone. For the more saturated, longer chain-length lipids, 40 mole percent cholesterol decreased half lives significantly over that observed for PC alone.

The effect of cholesterol and PG was examined in a similar type of study using MLVs composed of 50 mole percent of a selected PC, 10 mole percent of a selected PG, 40 mole percent of cholesterol, and 0.1 mole percent $\alpha$-T, as indicated at the left in Table 5. The drug encapsulation data for the eight MLV preparations and the corresponding drug efflux half lives are shown at the middle and right columns, respectively, in Table 5. As seen, the presence of 10 mole percent PG significantly increased the drug efflux half lives of each PC lipid with respect to the Table 4 lipid composition containing PC and cholesterol only.

Table 5

| PC/PG/CH/$\alpha$-T (5:1:1:0.1) | % Encapsulated MPS | $t_{1/2}$ (minutes) |
|---|---|---|
| SPC/EPG/CH | 10 | 326 |
| DOPC/DOPG/CH | 11 | 235 |
| EPC/EPG/CH | 12 | 250 |
| DMPC/DMPG/CH | 10 | 591 |
| DPPC/DPPG/CH | 12 | 941 |
| HEPC/EPG/CH | 13 | 334 |
| HSPC/EPG/CH | 12 | 553 |
| DSPC/DSPG/CH | 27 | 1148 |

Finally, eight liposome preparations containing one of the PC lipids indicated at the left in Table 6 (60 mole percent); cholesterol hemisuccinate, a negatively charged sterol (40 mole percent); and 0.1 mole percent $\alpha$-T were prepared as above. CHHS was obtained from Sigma Chem Co. (St. Louis, MO). The eight preparations gave the encapsulation efficiencies and drug efflux half lives indicated in the middle and right columns, respectively, in Table 6 below. As seen, the presence of 40 mole percent cholesterol hemisuccinate gave uniformly high encapsulation efficiencies. The half lives are generally somewhat greater than those observed with the corresponding liposome preparations involving 40 mole percent cholesterol (Table 4 above), but significantly less than the corresponding lipid preparations containing 40 mole percent cholesterol plus 10 mole percent PG.

Table 6

| PC/CHHS/$\alpha$-T (9:1:0.1) | % Encapsulated MPS | $t_{1/2}$ (minutes) |
|---|---|---|
| SPC/CHHS | 19 | 73 |
| DOPC/CHHS | 19 | 123 |
| EPC/CHHS | 24 | 77 |
| DMPC/CHHS | 20 | 129 |
| DPPC/CHHS | 18 | 245 |
| HEPC/CHHS | 20 | 601 |
| HSPC/CHHS | 19 | 856 |
| DSPC/CHHS | 23 | 768 |

Example IV

Aerosolizing Aqueous Liposome Suspensions

This section examines the effect on liposome integrity, as judged by loss of encapsulated material and change in liposome size, of aerosolizing an aqueous liposome suspension.

Four liposome preparations were tested. Preparation #1 had a composition EPC/CH/$\alpha$-T, at a molar ratio of 6:4:0.1; preparation #2, a composition of DSPC/CH/$\alpha$-T, at a molar ratio of 6:4:0.1; preparation #3, a composition of DPPC/DPPG/CH/$\alpha$-T, at a molar ratio of 5:1:4:0.1; and preparation #4, a composition of DPPC/DPPG/$\alpha$-T, at a molar ratio of 9:1:0.1. The four 8% for the liposome preparation having the lowest efflux half life to about 1% for the material having the highest efflux half life. No significant change in the size distribution of liposomes was observed for any of the four liposome preparations after aerosolization.

15

EP 0 223 831 B1

## Table 7

| Preparation | | % Encap. $\pm$ s.d. (n=3) | Median Lip. size (nm) |
|---|---|---|---|
| 1 | B | $96.2\pm0.11$ | 214, 210-218 |
| $t_{1/2} =$ | A | $88.5\pm0.13$ | 375, 315-428 |
| 206 min. | R | $95.9\pm0.04$ | 193, 189-200 |
| 2 | B | $95.5\pm0.20$ | 230, 214-268 |
| $t_{1/2} =$ | A | $93.0\pm0.08$ | 200, 180-240 |
| 421 min. | R | $96.7\pm0.13$ | 200, 190-218 |
| 3 | B | $97.2\pm0.63$ | 327, 271-450 |
| $t_{1/2} =$ | A | $93.4\pm0.04$ | 257, 225-327 |
| 941 min. | R | $96.2\pm0.93$ | 353, 300-400 |
| 4 | B | $96.1\pm0.19$ | 214, 193-231 |
| $t_{1/2} =$ | A | $95.1\pm0.11$ | 218, 171-266 |
| 5256 min. | R | $96.5\pm0.02$ | 225, 200-276 |

The effect on the integrity of the four liposome preparations of aerosolization by an ultrasonic nebulizer was also examined. The nebulizer used was a DeVilbiss Pulmasonic Model 25 Ultrasonic Nebulizer (Somerset, PA), operated at a power mode of 3 kHz. The percent carboxyfluorescein encapsulation and size distribution, measured as above, are shown in Table 8 below for each of the four preparations, measured before (B) and after (A) aerosolization and for material remaining in the nebulizer (R). The results are substantially identical with the results obtained for the same preparations aerosolized by a compressed-air device.

16

## Table 8

| Preparation | | % Encap.±s.d.(n=3) | Median Lip. size (nm) |
|---|---|---|---|
| | B = Before | A = After | R = Remainder |
| 1 | B | 95.6±0.10 | 316, 285-375 |
| | A | 89.4±0.65 | 261, 250-279 |
| | R | 94.9±0.45 | 207, 188-222 |
| 2 | B | 98.6±0.08 | 240, 185-300 |
| | A | 96.8±0.07 | 214, 200-240 |
| | R | 97.7±0.53 | 163, 150-189 |
| 3 | B | 98.1±0.09 | 257, 240-270 |
| | A | 96.0±0.09 | 254, 244-270 |
| | R | 97.2±0.10 | 240, 171-600 |
| 4 | B | 98.5±0.02 | 333, 310-375 |
| | A | 96.2±0.01 | 220, 204-231 |
| | R | 96.8±0.03 | 220, 208-230 |

Example V

Liposome Stability in Fluorocarbon Propellants

This section examines the stability of three liposome preparations in chlorofluorocarbon propellant solvents. The first liposome preparation, referred to as formulation 1, contained DPPC/DPPG/$\alpha$-T at a molar ratio of 9:1:0.1; the second preparation, designated formulation 2, contained EPC/EPG/$\alpha$-T at a molar ratio of 9:1:0.1; and the third preparation, designated formulation 3, was composed of EPC/EPG/CH/$\alpha$-T at a molar ratio of 5:1:4:0.1. The liposomes were prepared substantially as described in Example I, except that aqueous solution used in forming the MLVs contained bovine serum albumin (BSA) and 1 $\mu$M carboxyfluorescein (CF). MLV preparations extruded twice through each of a 0.4 and a 0.2 $\mu$m (micron) polycarbonate membrane were pelleted by centrifugation to remove unencapsulated material, and resuspended in PBS to a final lipid concentration of about 20 $\mu$mol/ml. The six propellant solvents used are "Freon 11" ($CCl_3F$), "Freon 12" ($CCl_2F_2$), "Freon 22" ($CHClF_2$), "Freon 113" ($CCl_2FCClF_2$), "Freon 114" ($CClF_2CClF_2$), and "Freon 115" ($CClF_2CF_3$).

An aqueous suspension of each preparation in PBS (3 ml) was added to each of the 6 fluorocarbon solvents (10 ml) in a pressurized vial, and the vials were shaken on a mechanical shaker for 24 hours to maintain a water-and-oil emulsion. After the shaking period, the bulk liquid phases in each vial were allowed to reform by settling, and the upper aqueous phase was removed by aspiration. The liposomes in the aqueous phase were pelleted by centrifugation, and resuspended to the original lipid concentration in PBS. A portion of the resuspended liposomes was examined for particle size distribution, substantially as described in Example IV. A control sample, which was not exposed to a fluorocarbon but was otherwise handled identically to the other samples, showed a size distribution of between about 180 and 250 nm. The various samples which had been exposed to the six propellant solvents generally fell into two size ranges: those having sizes substantially identical to the control liposome sizes, i.e., between about 150 and 300 nm;

and those having predominantly greater sized (400-500 nm or larger). The results, seen in Table 9 below, show that two of three preparations were stable in "Freon 12", that all three were stable in "Freon 114" and "Freon 115", and that MLVs containing largely saturated lipids (formulation 1) are generally more stable in fluorochlorocarbon propellants than those having predominantly unsaturated and/or shorter-chain phospholipids (formulations 2 and 3).

The pelleted and resuspended liposomes were also examined for release of BSA, representative of a relatively large molecular weight marker, and carboxyfluorescein, representative of a relatively small encapsulated marker. For each test, an aliquot of the resuspended liposomes were assayed by conventional methods for the appropriate marker. The marker concentrations are expressed in Table 9 in terms of amount marker per $\mu$mole of total lipid in the sample assayed ($\mu$g of BSA, $\mu$mole of CF). "ND" in the table means "not detectable ".

Table 9

| Freon Propellant | Formulation 1 | | | Formulation 2 | | | Formulation 3 | | |
|---|---|---|---|---|---|---|---|---|---|
| | Size | BSA | CF | Size | BSA | CF | Size | BSA | CF |
| Control | 191±26 | 14.3 | 11.4 | 248±84 | 7.1 | 3.8 | 248±60 | 8.2 | 5.9 |
| 11 | 171±35 | 13.5 | 12.6 | 495±293 | ND | ND | 560±355 | ND | ND |
| 12 | 177±44 | 11.4 | 10.3 | 231±76 | 8.2 | 3.2 | 703±400 | 4.0 | 1.9 |
| 22 | 1060±570 | ND | ND | 475±260 | 5.9 | 1.8 | 772±430 | 12.0 | 1.1 |
| 113 | 157±43 | 10.1 | 8.8 | 254±60 | ND | ND | 160±60 | ND | ND |
| 114 | 188±40 | 11.2 | 9.3 | 236±65 | 9.7 | 3.7 | 220±50 | 7.8 | 8.0 |
| 115 | 181±40 | 13.2 | 11.1 | 188±50 | 8.6 | 3.2 | 240±55 | 8.0 | 5.8 |

ND=not detectable

As seen from the data in the table, the loss of encapsulated material correlates generally with increased liposome size, both reflecting instability of liposomes in the particular fluorochlorocarbon solvent. However, both preparations 2 and 3 showed substantial loss of encapsulated material without significant size change when exposed to "Freon 113"..

Example VI

Treatment of Bronchoconstriction

EP 0 223 831 B1

This example examines the therapeutic effect of free and liposome-encapsulated metaproteranol sulfate on bronchoconstriction induced by aerosols of acetylcholine (ACH).

Two mongrel dogs, a 20 kg male and a 30 kg female, were anaesthetized with an initial i.v. bolus injection of sodium thiopental (10 mg/kg body weight) and sodium pentabarbital (20 mg/kg body weight) and subsequent boluses of sodium thiopental every thirty minutes, or as needed, to maintain the dog in an anaesthetized state over a four hour period. The animals were intubated with a cuffed endotracheal tube, placed on a heat-exchange pad and artificially ventilated by a constant-volume respirator with room air at a frequency of 30 cycles/minute. An esophageal balloon catheter was introduced into the esophagus, connected to one port of a differential pressure transducer, and positioned in the mid-thorax to produce a maximal negative pressure inflection during inspiration. Air flow was monitored by a pneumotachograph connected to the endotracheal tube and the respirator and connected to a differential pressure transducer. Transpulmonary pressure was monitored by a differential pressure transducer, one port connected to a side arm of the tracheal tube and the other end connected to the esophageal balloon catheter. The flow and pressure signals were displayed continuously on an oscilloscope.

Liposome preparations having a lipid composition of DSPC/DSPG/CH/$\alpha$-T at a molar ratio of 5:1:4:0.1 (Preparation 1) and EPC/EPG/CH/$\alpha$-T at the same molar ratio (Preparation 2), each encapsulating metaproteranol sulfate at a final 2% drug concentration, were prepared as in Example III. As seen from Table 5 above, Preparation 1 has a drug efflux half life of about 1150 minutes, and Preparation 2, of about 250 minutes. Free MPS was administered as a 2% solution in PBS. Liquid aerosols of acetylcholine solutions sufficient to produce transient bronchoconstriction, as reflected by a 2-10 fold increase in total pulmonary resistance were administered via the endotracheal tube before, and at roughly 15 minute intervals following, aerosol administration of either free or liposome-encapsulated metaproteranol, each in doses corresponding to a total of about 2.4 mgs MPS. All aerosls, including the acetylcholine aerosol, free-drug aerosol, and liposome aerosol, were generated with a pneumatic nebulizer (Bird #158, Somerset, PA), according to manufacturer's specifications, at 103 kPa (15 psi). Aerosol particle sizing performed with a Mercer Cascade Impactor (InTox Products, NM), indicated mass aerodynamic aerosol particle diameters within 2-6 $\mu$m (microns).

The effects of the free-drug and liposome aerosols on bronchoconstriction were measured approximately every fifteen minutes for four hours after initial drug administration. The experiment was repeated 3 times for each free and liposome-encapsulated drug formulation for each animal, and the values averaged. The results obtained for dog #1 are shown in Figure 1, and are expressed in terms of percent protection from ACH-induced bronchoconstriction. Each point on the graph represents the mean ± standard error of the mean from results obtained in three experiments. As seen from the figure, free drug (open squares), Preparation 1 (closed circles), and Preparation 2 (open circles) all showed substantially the same degree of protection against ACH-induced bronchoconstriction. If any trend is discernible from the data, it is that the liposome-encapsulated drug appears to be more effective in the period between 2-4 hours after drug administration. Similar results were obtained for the other dog treated.

Example VII

Systemic Drug Delivery by Inhalation

The systemic drug levels of MPS, when administered by inhalation in either free or liposome-encapsulated form, were examined by monitoring the change in heart rate produced by administration of the drug according to the experimental protocol described above in Example VI. For each animal study conducted in Example VI, heart rate measurements were taken approximately every fifteen minutes in the four-hour period following drug administration. The heart rate was measured continuously on an oscilloscope via skin-needle leads and recorded intermittently on a polygraph. The data obtained for each of three runs on each of the three drug preparations (free drug and drug encapsulated in long half-life and short half-life liposomes) were averaged and plotted as percent increase over normal resting heart rate. The results for one of the treated animals are shown in Figure 2. As in Figure 1, each point represents the mean ± standard error of the mean from results obtained in three experiments. As seen in the figure, the free drug produced a more than 90% increase in heart rate within the first five minutes of drug administration, and the measured heart rate declined gradually over the 4-hour course of the measurement period to a level of about a 50% heart rate increase. Preparation 1 (the long half-life liposomes) showed an initial heart rate increase of between about 20-30% and remained within that range substantially over the entire monitoring period. Preparation 2 (the short half-life liposomes) gave an initial heart rate increase of about 40%, and this level also remained substantially constant over the four-hour monitoring period.

20

## Claims

1. A system for administering a water-soluble bronchodilator drug to the respiratory tract, comprising liposomes containing more than 50% of the drug in liposome-encapsulated form for a selected drug release rate, and
   a device for aerosolizing a metered quantity of the liposomes, in a form suitable for inhalation.

2. The system of claim 1, wherein the liposomes are suspended in an aqueous medium, and said device is a pneumatic nebulizer.

3. The system of claim 1, wherein the liposomes are suspended in a chlorofluoro(hydro)carbon propellant, and said device includes a valve structure for releasing the suspension under pressure produced by the propellant.

4. The system of claim 3, wherein the propellant is selected from $CCl_2F_2$, $CCIF_2CCIF_2$, and $CCIF_2CF_3$.

5. The system of claim 4, wherein the liposomes are suspended in dehydrated particulate form in the propellant.

6. The system of claim 1, wherein the liposomes are in dehydrated, particulate form.

7. The system of claim 1, wherein the liposomes are suspended in an aqueous medium, and more than 70% of the drug is in liposome-encapsulated form.

8. The system of any one of the preceding claims, wherein the drug is metaproterenol or a pharmaceutically acceptable salt thereof.

9. The system of any one of the preceding claims, wherein the rate of drug release from the liposomes when administered to the respiratory tract is proportional to the drug-release rate in vitro, and the phospholipid composition of the liposomes is formulated to produce a selected in vitro drug efflux half-life of between 250 and 1200 minutes.

## Revendications

1. Système pour administrer un médicament bronchodilatateur soluble dans l'eau à l'appareil respiratoire, comprenant des liposomes contenant plus de 50 % du médicament sous une forme encapsulée dans les liposomes en vue d'une vitesse choisie de libération du médicament, et

   un dispositif pour pulvériser sous forme d'aérosol une quantité mesurée des liposomes, sous une forme appropriée à l'inhalation.

2. Système selon la revendication 1, dans lequel les liposomes sont en suspension dans un milieu aqueux, et ledit dispositif est un nébuliseur pneumatique.

3. Système selon la revendication 1, dans lequel les liposomes sont en suspension dans un propulseur chlorofluoro(hydro) carboné, et ledit dispositif comprend une structure de valve pour libérer la suspension sous la pression produite par le propulseur.

4. Système selon la revendication 3, dans lequel le propulseur est choisi parmi $CCl_2F_2$ , $CCIF_2CCIF_2$ , et $CCIF_2CF_3$.

5. Système selon la revendication 4, dans lequel les liposomes sont en suspension sous forme de particules déshydratées dans le propulseur.

6. Système selon la revendication 1, dans lequel les liposomes sont sous forme de particules déshydratées.

7. Système selon la revendication 1, dans lequel les liposomes sont en suspension dans un milieu aqueux, et plus de 70 % du médicament est sous une forme encapsulée dans les liposomes.

8.  Système selon l'une quelconque des revendications précédentes, dans lequel le médicament est le métaprotérénol ou l'un de ses sels pharmaceutiquement acceptables.

9.  Système selon l'une quelconque des revendications précédentes, dans lequel la vitesse de libération du médicament à partir des liposomes lorsqu'il est administré à l'appareil respiratoire est proportionnelle à la vitesse de libération du médicament in vitro, et la composition phospholipidique des liposomes est formulée pour produire une demi-vie de dégagement du médicament in vitro choisie entre 250 et 1200 minutes.

**Patentansprüche**

1.  System zur Abgabe eines wasserlöslichen bronchienerweiternden Arzneimittels an den Atemtrakt, welches Liposome enthält, wobei mehr als 50 % des Arzneimittels in liposomeingekapselter Form für eine selektive Arzneimittelabgaberate vorliegt und mit einer Vorrichtung zum Zerstäuben einer abgemessenen Menge der Liposome in für die Inhalation geeigneter Form.

2.  System nach Anspruch 1, in welchem die Liposome in wässrigem Medium suspendiert sind und in welchem die Vorrichtung ein pneumatischer Zerstäuber ist.

3.  System nach Anspruch 1, in welchem die Liposome in einem Chlor-fluor-kohlenwasserstofftreibmittel suspendiert sind und wobei die Vorrichtung eine Ventilvorrichtung zur Abgabe der Suspension unter durch das Treibmittel erzeugten Druck umfaßt.

4.  System nach Anspruch 3, in welchem das Treibmittel zwischen $CCl_2F_2$, $CClF_2CClF_2$ und $CClF_2CF_3$ ausgewählt wird.

5.  System nach Anspruch 4, in welchem die Liposome in wasserfreier Partikelform in dem Treibmittel suspendiert sind.

6.  System nach Anspruch 1, in welchem die Liposome in entwässertem, partikelförmigem Zustand eingesetzt werden.

7.  System nach Anspruch 1, in welchem die Liposome in einem wässrigen Medium suspendiert sind und in welchem mehr als 70 % des Arzneimittels in liposomeingekapselter Form vorliegt.

8.  System nach einem der vorhergehenden Ansprüche, in welchem das Arzneimittel Metaproterenol oder ein pharmazeutisch anwendbares Salz desselben ist.

9.  System nach einem der vorangehenden Ansprüche, in welchem die Arzneimittelabgaberate von den Liposomen proportional zur Arzneimittelabgaberate in vitro ist, wenn sie an den Atemtrakt abgegeben werden und wobei die Phospholipide der Liposome derart zusammengesetzt sind, daß sie eine ausgewählte in vitro Arzneimittelabgabehalbwertszeit von 250 bis 1200 Minuten erzeugen.

FIG. 1

Minutes post bronchodilator

FIG. 2

Minutes post bronchodilator

FIG. 3A

FIG. 3B

FIG. 4